(19) Europäisches Patentamt

European Patent Office

Office européen des brevets

(11) **EP 1 655 601 A1**

(12) **EUROPEAN PATENT APPLICATION**
published in accordance with Art. 158(3) EPC

(43) Date of publication:
**10.05.2006 Bulletin 2006/19**

(51) Int Cl.:
***G01N 22/04*** (1980.01)

(21) Application number: **04748118.9**

(22) Date of filing: **30.07.2004**

(86) International application number:
**PCT/JP2004/010957**

(87) International publication number:
**WO 2005/012887 (10.02.2005 Gazette 2005/06)**

(84) Designated Contracting States:
**AT BE BG CH CY CZ DE DK EE ES FI FR GB GR
HU IE IT LI LU MC NL PL PT RO SE SI SK TR**

(30) Priority: **31.07.2003 JP 2003284314**

(71) Applicant: **OJI PAPER CO., LTD.
Tokyo 104-0061 (JP)**

(72) Inventors:
• **NAGATA, Shinichi,
c/o OJI PAPER Co. Ltd.
Amagasaki-shi,
Hyogo 660-0811 (JP)**

• **SAWAMOTO, Hidetada,
c/o OJI PAPER Co. Ltd.
Amagasaki-shi,
Hyogo 660-0811 (JP)**

(74) Representative: **Zinkler, Franz et al
Schoppe, Zimmermann, Stöckeler & Zinkler
Postfach 246
82043 Pullach bei München (DE)**

(54) **METHOD AND DEVICE FOR MEASURING MOISTURE CONTENT**

(57)     A method of measuring moisture content, wherein, as an example, a microwave cavity resonator with two holed iris plates that are arranged vertically in tube axis at the mid point of a wave guide is used. A specimen is disposed in a slit formed at a resonator portion between the iris plates, and a resonance peak level is measured in real time. Based on a difference in resonance peak level between cases when the specimen is not present in the slit and the specimen is present in the slit, the moisture content or the moisture percentage of the specimen is obtained.

FIG. 1

## Description

FIELD OF THE INVENTION

The present invention relates to a method and a device used for measuring moisture content or moisture percentage of a sheet-material, such as paper, non-woven fabric, and film, by using resonance of microwaves.

BACKGROUND ART

Conventionally, with respect to the method of measuring the moisture content or moisture percentage of a sheet-shaped material, methods utilizing absorption of infrared rays and methods utilizing microwaves have been used. The method utilizing absorption of infrared rays tends to absorb infrared rays from an ambient heat source, and is consequently subjected to influences from disturbance heat, with the result that it sometimes fails to accurately measure the moisture content or moisture percentage. Moreover, the method utilizing absorption of infrared rays is considered to have difficulty in measuring a fine amount of moisture, and fails to measure the moisture content or moisture percentage of a sheet-shaped specimen such as a film on which an organic substance is applied.

The first example of the method utilizing microwaves is a method utilizing traveling waves. Such a method has been devised in which: a sheet-shaped specimen is placed so as to be made in contact with a microwave wave guide path, and as microwaves proceed through the wave guide path while being made in contact with the specimen, the power of the microwaves is gradually decreased by the moisture. For example, Japanese Examined Patent Publication No. 47-9080 describes a device that measures moisture content of a specimen by using a microwave hom antenna. However many inventions of this type have been applied.

However, the method in which the traveling waves of microwaves are utilized is different from a method utilizing resonance to which the present invention is directed. When microwaves come into contact with a specimen, return waves reflected from the specimen collide with traveling waves on the way to return to be composed into a standing wave; however, this wave is completely different from a standing wave resutting from resonance that relates to the present invention physically as well as on a basis of an electric circuit The standing waves derived from both reflection and resonance are similar to each other in that with respect to a certain fixed position on fixed coordinates, a position at which an electric field and a magnetic field are set to zero and a position at which those become greatest are unchanged; however, since the standing wave derived from reflection is not resonance, a resonance curve is not obtained. The resonance refers to a resonance state on an electric circuit basis, that is, three factors including inductance L, capacitance C and resistance R need to be included in a measuring system provided with a resonator, and a resonance condition that reactance components become zero at a resonance point needs to be satisfied.

The second example of the method utilizing microwaves is a method utilizing resonance. Japanese Examined Patent Publication No. 58-30534 describes a method in which moisture content is measured by using a cavity resonator. In this case, a cavity resonator is used in which: a pair of wave guides that are placed in a manner so as to sandwich a specimen, with flanges being respectively attached outward to openings thereof adjacent to the specimen, and these flanges are facing to be capacitive-coupled with each other.

Japanese Patent Application Laid-Open No. 62-238447 has proposed a method in which a resonance frequency and a peak voltage in a cavity resonator are measured so that both moisture and basis weight are calculated from a characteristic equation; however, no description has been given on the structure of the cavity resonator itself. The measuring method of Japanese Patent Application Laid-Open No. 62-238447 describes that, by finding that the moisture "X" and the basis weight "Y" do not devote to measured data of the frequency "f" and voltage "v" at the resonance peak level of microwaves in an independent manner respectively and that both of the moisture "X" and the basis weight "Y" are correlated with each other, both the moisture and basis weight can be calculated only by using a characteristic equation.

Japanese Patent Application Laid-Open No. 62-169041 has described a device in which a plurality of cavity resonators are arranged to measure moisture content; however, the cavity resonator used in this case is a re-entrant type cavity resonator. The re-entrant type cavity resonator does not regulate the electric field distribution, and its electric field vector is not made in parallel with the sheet face of a sheet-shaped specimen. For this reason, the volume of the specimen that interacts with the electric filed is small, failing to provide sufficient measuring accuracy.

Japanese Examined Patent Publication No. 6-58331 has also described a moisture measuring process using a cavity resonator, however, the cavity resonator used in this process has a convex portion at a position corresponding to a measuring portion of the cavity resonator.

Japanese Patent Application Laid-Open No.62-169041 and Japanese Examined Patent Publication No.6-58331 also illustrate a normal cavity resonator in which the openings of a pair of wave guides are placed so as to face a specimen as a prior-art device; however, the cavity resonator of this type has a low Q value, failing to provide measurements with high accuracy.

DISCLOSURE OF THE INVENTION

OBJECTIVE OF THE INVENTION

The objective of the present invention is to provide a method and a device which makes it possible to measure the moisture content or the moisture percentage of a sheet-shaped material such as paper and films with superior measuring sensitivity and high accuracy even in the case of a fine amount of moisture, without being influenced by disturbance such as a heat source.

MEANS FOR SOLVING PROBLEM

A method of measuring moisture content in accordance with the present invention also uses a microwave cavity resonator, however, the cavity resonator of the present invention is provided with two iris plates holed which are arranged vertically in the direction of a tube axis at mid points of a wave guide, so that a resonator portion is formed between the iris plates and a traveling wave portion is formed outside of each of the iris plates, and with a slit in which a specimen is disposed being placed in a manner so as to cross the resonator portion. Moreover, the measuring frequency is limited to a predetermined range between 1 to 25 GHz, and the moisture content or the moisture percentage of the specimen is determined based upon a difference in measured resonance peak levels between cases when the specimen is not present in the slit and the specimen is present in the slit

Moreover, the measuring device of the present invention includes: a microwave cavity resonator that is provided with two holed iris plates which are arranged vertically in the direction of a tube axis at mid points of a wave guide so that a resonator portion is formed between the iris plates and a traveling wave portion is formed outside of each of the iris plates, and with a slit in which a specimen is disposed being placed in a manner so as to cross the resonator portion; a microwave sweep oscillator which is connected to one of the pair of traveling wave portions and oscillates at a frequency in a predetermined range between 1 to 25 GHz; a microwave intensity receiver that is connected to the other of the pair of traveling wave portions; and a data processing device which, upon receipt of a signal from the microwave intensity receiver, detects a peak level, and determines the moisture content or the moisture percentage of a specimen based upon the difference in measured resonance peak level between cases when the specimen is not present in the slit and the specimen is present in the slit

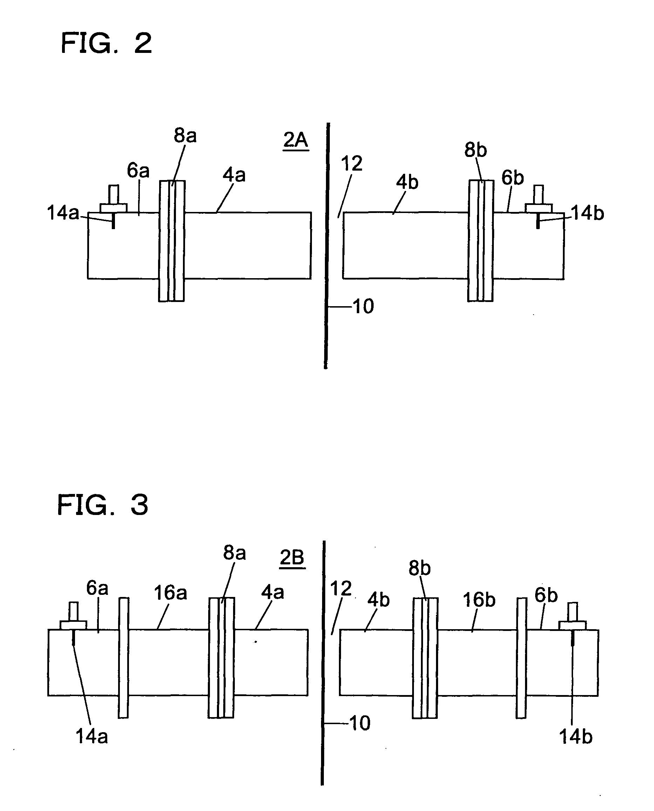

A microwave cavity resonator to be used in the present invention is shown in Fig. 2 or Fig. 3. The microwave cavity resonator shown in Fig. 2 has a wave guide 2A that is constituted by wave guide portions 4a, 4b, 6a and 6b. Two holed iris plates 8a and 8b are arranged vertically in the direction of a tube axis at mid points of a wave guide 2A. Resonator portions 4a and 4b are formed between the iris plates 8a and 8b, with the outside portions of the iris plates 8a and 8b respectively forming traveling wave portions 6a and 6b. The microwave cavity resonator has a slit 12 in which a specimen 10 is disposed being placed in a manner so as to cross the resonator portions 4a and 4b. A microwave sweep oscillator that oscillates at a frequency in a predetermined range between 1 to 25 GHz is connected to one of the traveling wave portions 6a, and a microwave intensity receiver is connected to the other traveling wave portion 6b. Reference numerals 14a and 14b respectively indicate antennas attached to the respective traveling wave portions 6a and 6b, and the antenna 14a is connected to the microwave sweep oscillator, while the antenna 14b is connected to the microwave intensity receiver.

The microwave cavity resonator shown in Fig. 3 has a wave guide 2B which is constituted by wave guide portions 4a, 4b, 4a, 4b, 16a and 16b. One of the paired traveling wave portions is constituted by a wave guide portion 16a adjacent to one of the iris plates 8a and another wave guide portion 6a, connected to this wave guide portion 16a, which is connected to the microwave sweep oscillator, and the other of the paired traveling wave portions is constituted by a wave guide portion 16b adjacent to the other iris plate 8b and another wave guide portion 6b, connected to this guide portion 16b, which is connected to the microwave intensity receiver.

The microwave cavity resonator in Fig. 3 is different from the microwave cavity resonator in Fig. 2 in that the wave guide portion 16a is further placed between the wave guide portion 6a of the traveling wave portion connected to the microwave sweep oscillator and the iris plate 8a, with the wave guide portion 16b being further placed between the wave guide portion 6b of the traveling wave portion connected to the microwave intensity receiver and the iris plate 8b. Both of the wave guide portions 16a and 16b correspond to the traveling wave portions.

In the present invention, microwaves are resonated in the resonator, and a specimen is placed in the resonator or in the vicinity of the resonator so that the moisture content or the moisture percentage contained in the specimen is measured from the resonance characteristic of the resonator system including the specimen.

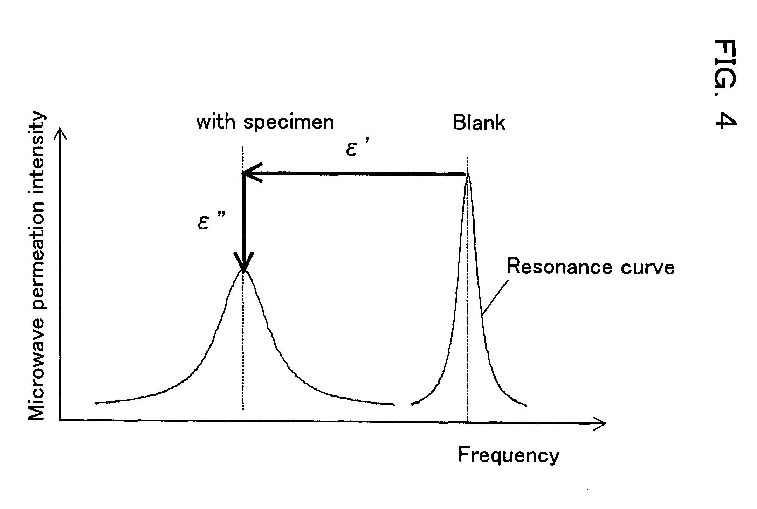

Upon resonance in the microwave resonator, a resonance curve as shown in Fig. 4 is obtained. The resonance curve on the right side is a resonance curve in a state without a specimen (blank). When a specimen is present inside the resonator or in the vicinity thereof, the resonance frequency is shifted toward the low frequency side as indicated by a resonance curve on the left side by a dielectric constant possessed by the specimen, while the peak level is lowered due to the dielectric loss factor, resutting in a reduction in Q value.

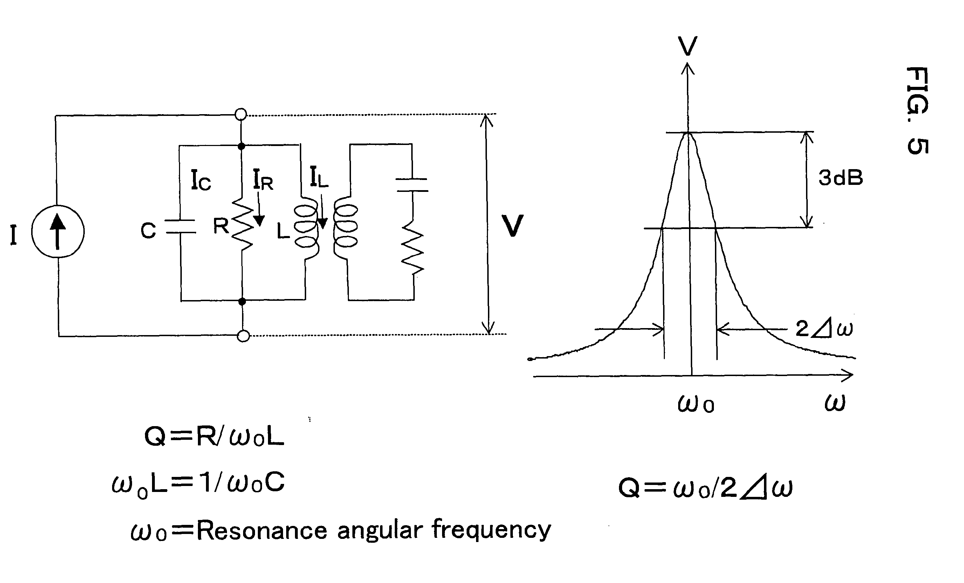

This phenomenon is explained through various methods, and as shown in Fig. 5, it is also explained by a theory of an LCR resonance circuit constituted by an inductance L, a capacitance C and a resistance R. In the cavity resonator of the present invention, the irises form a reactance component and a capacitance component, and a resistance component from the wave guide wall is added to this so that the LCR resonance circuit is formed. The dielectric constant of a specimen corresponds to the capacitance C and the dielectric loss factor corresponds to the resistance R so that these are equivalent to the coupling of L, C and R of the resonator through electromagnetic coupling. Therefore, when the specimen is present, the capacitance C increases, making the resonance frequency f (f= $1/\{2\pi (LC)^{1/2}\}$) smaller. Simultaneously, the resistance R increases due to the dielectric loss factor of the specimen while the peak level reduces, making the Q value smaller.

Moreover, since a cavity resonator is used in the present invention, the same phenomenon can also be explained by the perturbation theory, however, the explanation will be omitted here.

By taking into consideration that the peak level of a resonance curve changes due to this dielectric loss factor, the present invention measures the moisture content or moisture percentage.

Generally, the dielectric loss factor of a generally-used film is in the order of $10^{-4}$ to $10^{-2}$, while that of paper is in the order of $10^{-2}$ to $10^{-1}$. In contrast, the dielectric loss factor of water is 13.1 (at 3GHz), which is 100 times to 100000 times greater than that of the generally-used film and paper. In addition, in the case of the generally-used film, the dielectric loss factor is changed virtually only by moisture and crystallinity in a microwave range of, for example, about 4GHz, and with the exception of these, the other factors do not increase the dielectric loss factor of the film. The change in dielectric loss factor due to the crystallinity is actually dependent on the amount of absorption of microwave energy by molecular movements in a noncrystal portion (amorphous portion) of the film; therefore, for example, in the case of a PET (polyethylene terephthalate) film, the dielectric loss factor is in the order of $10^{-2}$ to $10^{-3}$, which is very small, and almost no change occurs as long as it is used under an environment having a temperature lower than the glass transition temperature (Tg). Consequently, if the dielectric loss factor of a generally-used film had changed, it is considered that the change is caused by moisture.

Based upon this idea, the inventors drew their attention to the dielectric loss factor and have devised a novel moisture content measuring method. In other words, they have devised a method of measuring the peak level of a resonance curve in real time, thereby making it possible to measure the moisture content of a sheet-shaped material such as a polymer film in an on-line state.

Through trial and error, the inventors have found that by using a method in which a microwave cavity resonator having iris plates as shown in Figs. 2 and 3 is used with the measuring frequency being selected to an appropriate range between 1 to 25 GHz as an optimal frequency range for microwaves to be used, measurements with higher accuracy are achieved stably in comparison with the conventional method of measuring the amount of damping in the traveling wave, and the peak level of a resonance curve can be found positively so that it becomes possible to carry out stable measurements without being influenced by the dielectric constant, that is, the capacitance of the specimen.

The method and device of the present invention will be compared with those of devices using a conventional microwave cavity resonator.

In measurements by using a microwave cavity resonator, when a specimen is inserted in the microwave cavity resonator, the resonance frequency must always be shifted toward the low frequency side due to a dielectric constant of the specimen. However, Japanese Examined Patent Publication No. 58-30534 describes that the resonance frequency is shifted toward the high frequency side. This description is completely opposite to that of the present invention in understanding on the principle on the relationships among the dielectric constant, dielectric loss factor and resonance curve, and judging from the principle of resonance, it is impossible for the resonance frequency to shift toward the higher side as a result of insertion of a specimen, in comparison with the state in which no specimen is present (in the case of negative dielectric constant, it is shifted toward the higher side; however, the dielectric constant is always positive). If the description of Japanese Examined Patent Publication No. 58-30534 is correct, its principle and basic ideas are completely different from those of the present invention.

Moreover, the mechanism of Japanese Examined Patent Publication No. 58-30534 that allows the resonator to resonate is different from that of the present invention. That is to say, Japanese Examined Patent Publication No. 58-30534 describes that "flanges $12_2$ and $13_2$ facing each other are respectively attached to openings $12_1$ and $13_1$ outwardly, and these flanges $12_2$ and $13_2$ are capacitive-coupled to each other to form a cavity resonator 11 (see lines 1 to 5 from the bottom of the 2nd column)"; however, the cavity resonator used in the present invention requires no flanges, and the mechanism forming the cavity resonator and the constituent elements are different from those of the reference. The cavity resonator of the present invention is characterized in that, as shown in Fig. 2, a wave guide is used, with plates having holes referred to as irises being inserted in the mid portions. The portion from an outer end to each of the irises corresponds to a traveling wave portion, and a portion between the irises corresponds to a cavity resonator, that is, a standing-wave portion. Here, the irises form a reactance component and a capacitance component, and a resistance component from the wave guide wall is added to this to form an LCR resonance circuit, which basically makes the present invention different from the cavity resonator of Japanese Examined Patent Publication No. 58-30534.

Japanese Patent Application Laid-Open No. 62-238447 also discloses a method in which both the moisture and basis weight are calculated based upon a characteristic equation using the resonance frequency and the peak voltage measured by a cavity resonator. However, this idea indicates that the moisture and the basic weight can be respectively calculated only by using the characteristic equation based on finding the fact that the moisture "X" and the basis weight "Y" do not devote to measured data of the frequency "f" and voltage "v" at the resonance peak level of a microwave in an independent manner respectively and that the moisture "X" and the basis weight "Y" correlate with each other. In contrast, the present invention is formed based upon the basic ideas that the resonance frequency is shifted toward the lower side due to a dielectric constant of a specimen and that the peak level is shifted toward the lower side due to a dielectric loss factor of the specimen. Here, it is noted that the dielectric loss factor of water is 13.1 (frequency: 3GHz), which is 10000 times to 100000 times greater than that of a film that is in the order of $10^{-3}$ to $10^{-4}$. The other factor except for moisture that makes the dielectric loss factor greater is only the dielectric loss due to molecular movements in amorphous parts of polymer, which is in the order of $10^{-3}$ and very small, causing no problems. Therefore, there is a basic idea that the moisture content can be measured by taking only the dielectric loss factor into consideration, and this idea makes the present invention basically different from Japanese Patent Application Laid-Open No. 62-238447.

Japanese Patent Application Laid-Open No. 62-169041 describes that a plurality of cavity resonators are arranged so as to measure the moisture content; however, the cavity resonators used in this case are re-entrant type cavity resonators, which are different from the cavity resonator provided with iris plates that is used in the present invention. The re-entrant type cavity resonator does not regulate the electric field distribution and is different from the present invention in that its electric field vector is not made in parallel with the sheet face of a sheet-shaped specimen. When the electric field is made in parallel with the sheet face as in the case of the present invention, the volume of the specimen that interacts with the electric field becomes greater, making it possible to greatly improve the measuring accuracy in comparison with the case in which the electric field and the sheet are perpendicular to each other, however, the re-entrant type cavity resonator fails to achieve such effects.

Japanese Examined Patent Publication No. 6-58331 also describes moisture measurements by the use of a cavity resonator, however, a cavity resonator in which a convex portion is formed at a position corresponding to a measuring portion is used, and any of the structure, electric field distribution and resonance mode are different from those of the cavity resonator of the present invention. As described in the Patent Document, this structure is adopted since it is difficult to actually form a complete rectangular parallelepiped structure; however, the structure is completely different from the structure of the present invention in which no convex portion is formed inside the wave guide.

In the present invention, the moisture content or moisture percentage of a specimen may be determined based upon a value obtained by dividing the difference in resonance peak level between the presence and absence of the specimen by a difference in resonance frequency between the presence and absence of the specimen. Measurements are carried out on a specimen of a film substrate bearing a coat layer on the surface thereof as well as on a specimen of a film substrate without the coat layer, and the moisture content or the moisture percentage of only the coat layer can be determined by subtracting the measured value of the specimen without the coat layer from the measured value of the specimen with the coat layer.

In the same manner, measurements are carried out on a specimen of a film substrate bearing a plurality of coat layers laminated on the surface thereof as well as on a specimen of a film substrate bearing coat layers except for the outermost coat layer of the coat layers, and the moisture content or the moisture percentage of only the outermost coat layer can be determined by subtracting the measured value of the specimen without the outermost coat layer of the coat layers from the measured value of the specimen with the plurality of coat layers.

In the microwave cavity resonator, it is preferred that holes of the iris plates are positioned on a tube axis. With respect to the holes of the iris plates, as the diameter thereof becomes larger, the microwave permeation intensity becomes greater, while the Q value becomes smaller. In contrast, as the diameter thereof becomes smaller, although the Q value becomes greater, the microwave permeation intensity becomes smaller. From the viewpoint of measuring the moisture content or the moisture percentage of a specimen, the hole diameter of each of the iris plates is appropriately set in a range from 1 to 20 mm.

The appropriate hole diameter of each iris plate is different depending on the frequency bands of microwaves. For example, with respect to a 4GHz band, the hole diameter is appropriately set in a range from 10 to 13 mm, and in this case, the $Q_0$ value (Q value without any specimen) is about 6000. With respect to a 12GHz band, the hole diameter is appropriately set in a range from 3.5 to 4.0 mm, and in this case the $Q_0$ value is about 5400. Moreover, with respect to a 19GHz band, the hole diameter is appropriately set in a range from 2.2 to 2.6 mm, and in this case the $Q_0$ value is about 4400.

The dimension of the resonator portion of the microwave cavity resonator is preferably set to a dimension at which the resonance mode is indicated by $TE_{10n}$, (n = 1, 2, 3, ...), and the slit in which a specimen is placed is preferably disposed at a position that allows the electric field vector to have the maximum value. The dimension of the resonator portion is dependent on the frequency of microwaves to be used for measurements, and for example, it is appropriately set in a range of 5 to 30 cm. For example, in the case of the microwave frequency of 4GHz, when a gap (width of a slit in which a specimen is placed) is 4 mm under $TE_{103}$ mode, the resonator portion is appropriately set to a dimension of 147.2 mm, and in the case of the microwave frequency of 12GHz, when a gap is 4 mm under $TE_{105}$ mode, the resonator portion is appropriately set to a dimension of 91.6 mm.

Both the wave guide to which the microwave sweep oscillator is connected and the wave guide to which the microwave intensity receiver is connected may be prepared as coaxial wave guide converters with one-sided flanges. Since such commercially available coaxial wave guide converters with one-sided flanges can be used, this structure can be easily achieved.

The measuring device of the present invention may be further provided with a specimen supplying mechanism for continuously supplying specimens to the slit of a microwave cavity resonator so as to become an on-line measuring device that continuously measures the specimens.

The present invention may be designed to have a structure in which: the temperature dependency of the resonance peak level has been preliminarily measured, a temperature of the microwave cavity resonator, a temperature of the surroundings of the slit or a temperature of the specimen when measuring the specimen is detected, and the measured resonance peak level value is corrected based upon the temperature dependency by using the detected temperature.

In order to carry out such a temperature correction, the moisture content measuring device of the present invention may be further provided with: a temperature dependency value storage unit that stores a preliminarily measured temperature dependency of the resonance peak level; and a temperature sensor which detects a temperature of the microwave cavity resonator, a temperature of the surroundings of the slit or a temperature of the specimen when measuring the specimen. Further, the data processing device may be provided with a correction means that corrects the resonance peak level measured value based upon the temperature dependency stored in the temperature dependency value storage unit by using the detected temperature from the temperature sensor.

If such an on-line measuring device is formed, it is preferable to prepare measures for preventing a specimen and the microwave cavity resonator from coming into contact with each other. With respect to the measures for preventing the specimen from coming into contact with the microwave cavity resonator when the specimen is directed to the slit, a guide having a specific shape for guiding the specimen to the slit may be attached to an end portion of the slit side on the outside face of an E face of the wave guide of the microwave cavity resonator.

With respect to the measures for preventing the specimen from coming into contact with the microwave cavity resonator when the specimen is moving through the slit, proximity sensors, which are placed in the vicinity of the slit at opposing positions with respective gaps to the surface and rear face of the specimen that passes through the slit, and a fluctuation detecting unit, which detects a fluctuation of the specimen that passes through the slit from the detected values of the proximity sensors, may be installed. Further, a save mechanism, which keeps away the microwave cavity resonator from the specimen when a detected fluctuation width obtained from the fluctuation detecting unit exceeds a predetermined reference value, may be further installed.

EFFECT OF THE INVENTION

In the measuring method and device in accordance with the present invention, a microwave cavity resonator provided with iris plates is used and the measuring frequency of microwaves to be used is set in a predetermined range between 1 to 25GHz so that the moisture content or the moisture percentage of a specimen is measured based upon a difference between a resonance peak level with a specimen and a resonance peak level without a specimen; therefore, it becomes possible to measure the moisture content or the moisture percentage of a sheet-shaped material with superior measuring sensitivity and high accuracy even in the case of a fine amount of moisture, without being influenced by disturbance such as a heat source.

In the case that the moisture content is determined based upon a value obtained by dividing a difference in resonance peak levels between the presence and absence of the specimen by a difference in resonance frequencies between the presence and absence of the specimen, specimens having various different thicknesses can be measured without having to measure the thickness of each of the specimens in a separate manner.

Among moisture content measuring devices, with respect to a method that has been realized as a so-called on-line measuring device that allows measurements of a specimen during its travel, hardly any method except for a method using infrared-ray absorption has been found. This method using the infrared-ray absorption is difficult to use for measuring a film-shaped specimen; in contrast, the present invention makes it possible to carry out on-line measurements on a film-shaped specimen.

BEST MODE FOR CARRYING OUT THE INVENTION

Fig. 1 shows a schematic structure of a moisture content measuring device in accordance with one embodiment;

Fig. 6 shows a schematic block diagram that indicates flows of signals; and Fig. 7 is a time chart

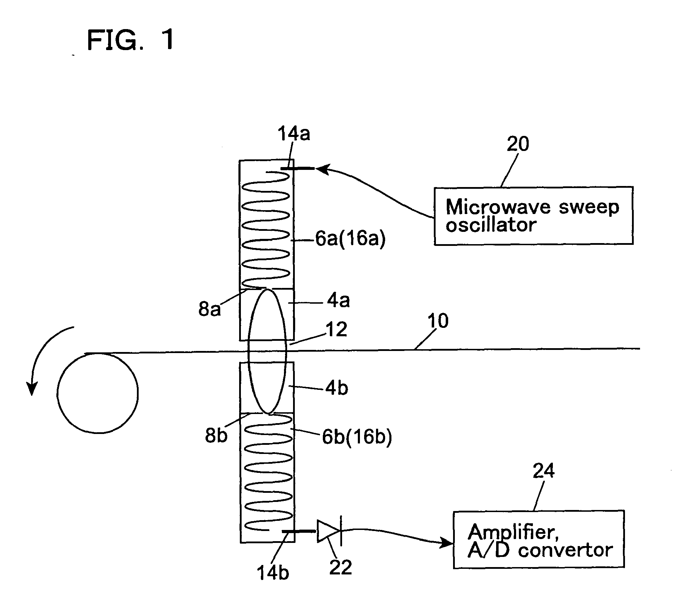

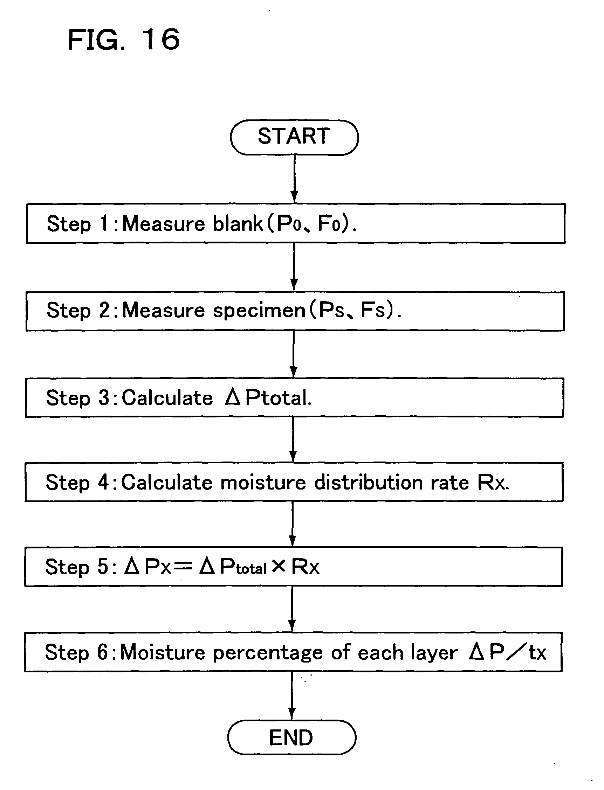

A specimen 10 is allowed to travel in contact with or in the vicinity of a microwave cavity resonator 1 so that a resonance peak level is measured in real time. This microwave cavity resonator 1 has a structure shown in Fig. 2 or Fig. 3, and as schematically shown in Fig. 1, two holed iris plates 8a and 8b are arranged vertically on a tube axis at the mid point of a wave guide. Each of these iris plates 8a and 8b has one hole positioned on the tube axis of the wave guide. Resonator portions 4a and 4b are formed between the iris plates 8a and 8b, and a slit 12 in which a specimen 10 is placed is formed in a manner so as to cross the resonator portions 4a and 4b. The outside portions 6a and 6b (in the case of Fig. 3, 16a and 16b are included) of the iris plates 8a and 8b form traveling wave portions. An exciter antenna 14a is attached to one of the traveling wave portions 6a, and a microwave sweep oscillator 20 that oscillates at a frequency in a predetermined range between 1 to 25GHz is connected to the antenna 14a. An antenna 14b is attached to the other traveling wave portion 6b, and a microwave intensity receiver constituted by a detector 22, an amplifier and A/D (analog/digital) converter 24 is connected to the antenna 14b.

The amplifier and A/D converter 24 of the microwave intensity receiver is connected to a data processing device. The data processing device includes a peak level detector 30 which, upon receipt of a signal from the amplifier and A/D converter 24, detects a resonance peak level, a resonance frequency detector 32 that detects a resonance frequency and an operation unit 34 that determines the moisture content or moisture percentage.

The operation unit 34 determines the moisture content or moisture percentage of a specimen 10 based upon a difference in resonance peak levels between cases when the specimen is not present in the slit 12 and the specimen 10 is present therein.

Moreover, the operation unit 34 may determine the moisture content of the specimen based upon a value obtained by dividing the difference in resonance peak level between the presence and absence of the specimen by a difference in resonance frequency between the presence and absence of the specimen.

Microwaves, released from the microwave sweep oscillator 20, are directed to the resonator portions 4a and 4b through the hole of the iris plate 8a so that resonance occurs in the resonator portions 4a and 4b, and one portion thereof is transmitted to the other antenna 14b through the hole of the iris plate 8b so that the resonance level is detected. When a specimen 10 is placed in the slit 12 between the resonance portions 4a and 4b, or placed close thereto, the resonance frequency is changed in response to the dielectric constant of the specimen 10, and the peak level changes in response to the dielectric loss factor.

The resonance level detected by the antenna 14b is converted to voltage by a detector diode 22. Thereafter, the voltage is directed to a peak detection circuit of a data processing device after having been subjected to amplifying and A/D converting processes in the amplifier and A/D converter 24. During a sweeping process, the peak detection circuit acquires 100,000 units of data, and simultaneously detects the maximum value (peak level) and the resonance frequency. This is repeated about every 50 milliseconds. In actual measurements, since the peak level tends to fluctuate due to various noises, averaging processes may be carried out so as to conduct stable measurements.

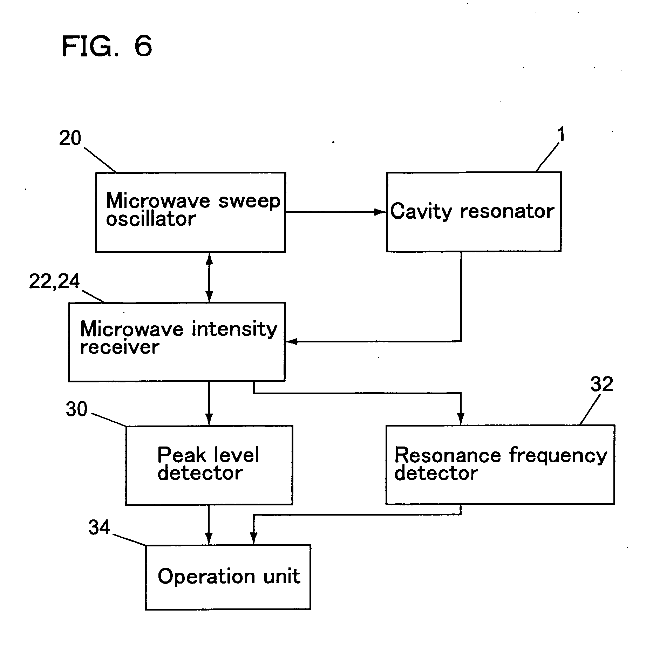

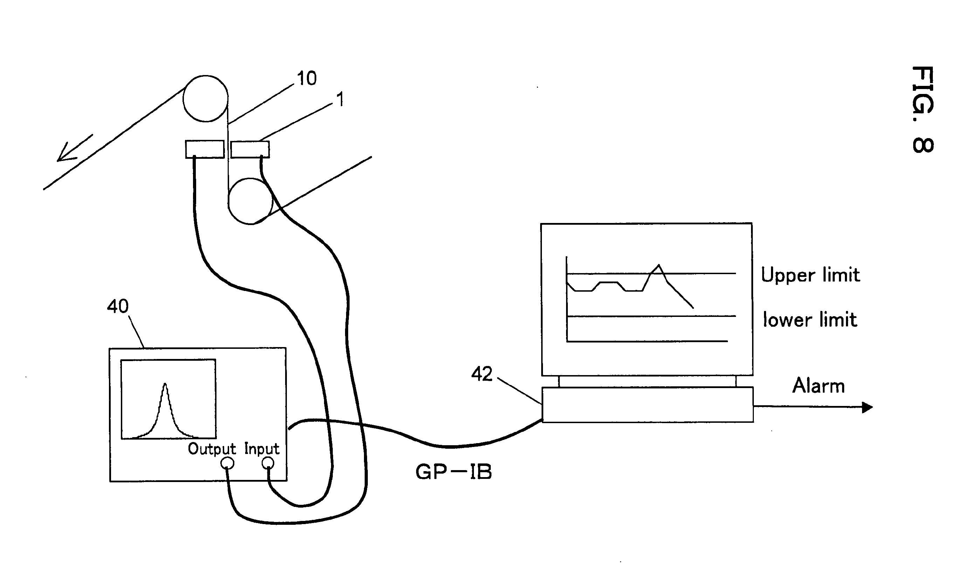

As shown in Fig. 8, as one example used for realizing the structure of Fig. 6, the cavity resonator 1 and a vector network analyzer 40 are connected to each other so that the peak level of a resonance curve in $S_{21}$ mode is sent to a personal computer 42 as data through a GP-IB interface; thus, moisture is measured in real time. By continuously supplying the specimen 10 through the supplying mechanism, an on-line measuring device is achieved.

Upon measuring the moisture content of a specimen of a sheet-shaped material such as a film and paper by using such an on-line measuring device, when the cavity resonator is shifted to a measuring position with the specimen being placed in the slit of the cavity resonator from a save position apart from the specimen, problems tend to arise in which the specimen fails to properly enter the slit portion inside the cavity resonator due to irregular movements (fluctuations perpendicular to the face) of the specimen during travel or curling (turning over) of the end portion to come into contact with the wave guide portion and in which the specimen is damaged by coming into contact with the wave guide end portion due to irregular movements.

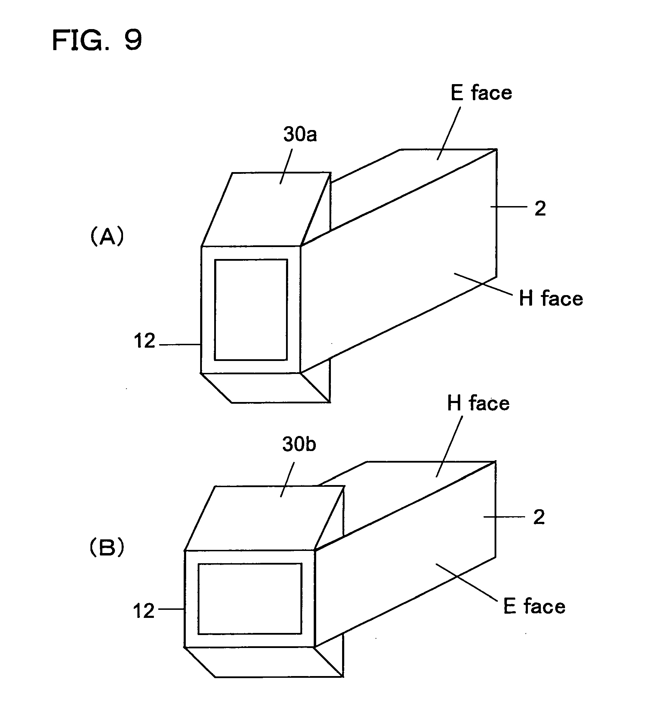

In order to solve these problems, as shown in Figs. 9(A) and 9(B), guides 30a and 30b made from resin are attached to the end portions of the slit 12 side of the wave guide 2. With this arrangement, for example, even when a specimen does not have a flat shape, the specimen first comes into contact with the guides 30a and 30b, and as the wave guide 2 further proceeds, the specimen is directed to the slit 12 along the guides 30a and 30b. With this arrangement, when the wave guide moves from the save position toward the measuring position, the specimen is smoothly guided into the slit even if the end portion of the specimen is curled. Moreover, even when the sheet-shaped specimen has irregular movements while traveling, the specimen is appropriately guided by the guides to be set in the slit

With respect to resins used for these guides 30a and 30b, those resins having a dielectric constant as small as possible, such as polyethylene, are preferably used so as to make microwaves hardly reflected.

With respect to positions at which the guides 30a and 30b are attached, the ends of the guides are preferably attached to the ends of wave guides, and it is necessary to design the arrangement so that the attached guides 30a and 30b do not cause a reduction in the Q value and resonance peak level. In result of experiments, it has been found that the attaching positions are preferably determined, not on an H face as shown in Fig. 9(B), but on an E face as shown in Fig.

9(A). Here, the E face refers to a face in parallel with an electric field inside the wave guide, and the H face refers to a face in parallel with a magnetic field inside the wave guide. The results can also be explained theoretically.

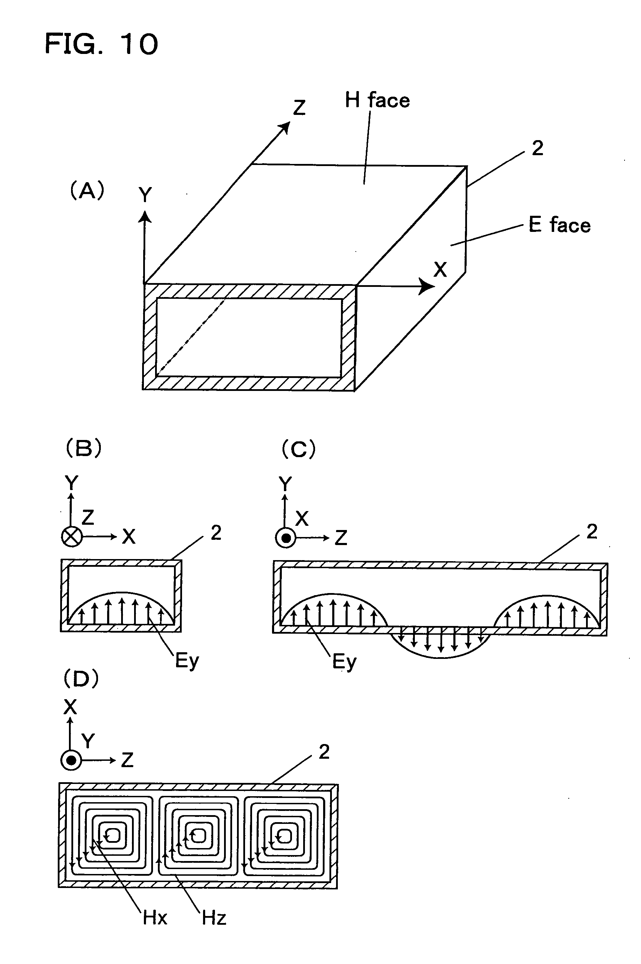

Figs. 10(B) to 10(D) show electromagnetic field distributions inside the cavity resonator in the case of $TE_{103}$ mode using a rectangular wave guide. Fig. 10(A) is a perspective view that shows a section of the cavity resonator taken in a direction perpendicular to the Z axis, and indicates the E face, H face, as well as X, Y and Z directions. Fig. 10(B) is a sectional view taken perpendicularly to the Z axis, Fig. 10(C) is a sectional view taken perpendicularly to the X axis, and Fig. 10(D) is a sectional view taken perpendicularly to the Y axis; and Ey indicates an electric field, and Hx and Hz indicate magnetic fields. At each of the end portions, the electric field (Ey) and magnetic fields (Hx, Hz) are distributed as shown in the Figures, and when resin is attached to the H face, the inside electric field intensity becomes stronger (the maximum in the center of the lateral direction), with one portion externally leaking, with the result that a resonance state is disturbed due to an interaction between the leak electric field and the resin to cause absorption or reflection and the subsequent reduction in peak level or reduction in Q value. In the case in which a resin guide is attached to the E face and in the case in which it is attached to the H face are compared with the case in which no guide is attached, the results as shown in Table 1 are obtained.

[Table 1]

|  | Attached to E face | Attached to H face | Without guide |
|---|---|---|---|
| Q value | 2738 | 1473 | 2745 |
| Peak level | -29.73 dB | -34.25 dB | -29.58 dB |
| Resonance frequency | 1249180 GHz | 1249000 GHz | 1249200 GHz |

The above-mentioned results clearly indicate that, in comparison with a case in which the guide is attached to the H face, when the guide is attached to the E face, it becomes possible to make reductions in the Q value and the peak level smaller.

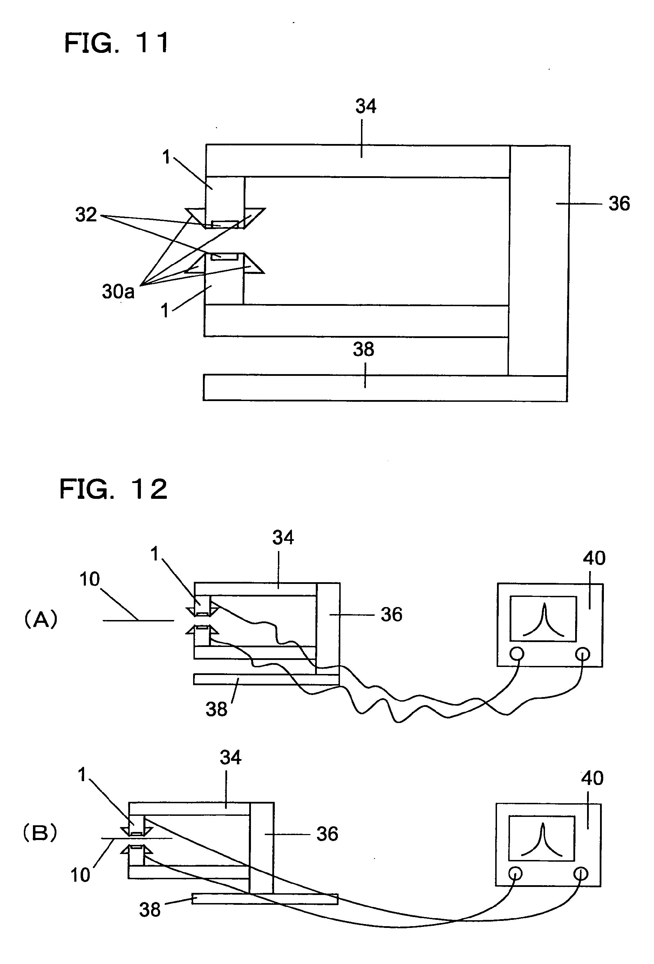

Moreover, depending on conditions of a specimen and the traveling speed thereof, the specimen fluctuates to be made in contact with the wave guide portion of the cavity resonator 1 inside the slit 12 of the cavity resonator 1. In order to solve the problem, a mechanism is prepared as shown in Fig. 11, in which two distance sensors 32 used for measuring the distance to a specimen are installed at the wave guide in a manner so as to sandwich the specimen, and when an irregular fluctuation exceeds a reference value, the measuring head is allowed to readily retreat to a save position. In this example, the cavity resonator 1 is supported by arms 34 and 36, and the arm 36 is supported so as to move along a slide rail 38 between the save position shown in the Figure and the measuring position at which the specimen is interpolated in the slit 12.

Fig. 12 schematically shows the entire measuring system. (A) shows a save position and (B) shows a measuring position. A cavity resonator 1, constituted by a pair of wave guides, corresponds to a measuring head, and this is supported by arms 34 and 36 so that this arm 36 is allowed to alternately move between the measuring position (B) and the save position (A) through a sliding mechanism.

This mechanism may be further revised to design the arms 34 and 36 so that the head measuring position is moved over the entire region in the width direction of the specimen. With this arrangement, the positional distribution of moisture content of the specimen in the width direction can be measured.

The circuit which receives a signal of the distance sensor 32 is designed so that it always releases a digital signal by comparing the measured distance with a reference level. When the fluctuation of the specimen is greater than the reference value, a solenoid valve in the mechanism for moving the arm 36 is turned on upon receipt of the digital signal from the circuit so that the arm 36 is allowed to move through an air force along the slide rail 38 to return to the save position at a high speed.

Conventionally, the specimen was made in contact with the wave guide portion due to irregular movements of the specimen to incur damages such as scratches; however, this method makes the specimen virtually free from scratches, making it possible to carry out moisture content measurements in a stable manner.

Two coaxial cables are connected so as to measure the peak level of the cavity resonator 1 in real time, for example, by using a network analyzer 40 so that the measured value is taken in a computer from the network analyzer 40 in real time.

Upon carrying out an on-line measurement, dust, dust particles and the like tend to adhere to the end portion and inside of the wave guide portion of the cavity resonator to cause adverse effects to the measurement In order to prevent such an adhesion, air pipes, nozzles, etc. are preferably attached to arms 34 and 36 so that air is regularly blown from the end portion toward the inside of the wave guide portion before and after the measuring process. The nozzles are preferably placed with a slight distance from the wave guide so as to reduce adverse effects given to the measurement

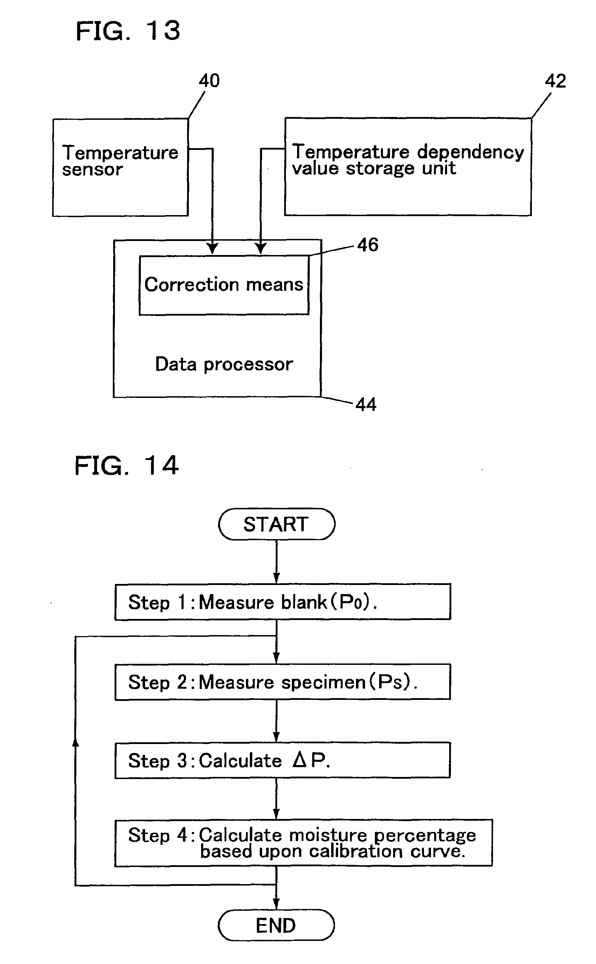

The resonance peak level changes depending on the temperature. For this reason, it is preferable to correct the peak level based on the temperature change. Therefore, as shown in Fig. 13, a temperature dependency value storage unit 42 that stores the temperature dependency, which has been measured preliminarily, and a temperature sensor 40 that detects upon measuring a specimen the temperature of the surroundings of the microwave cavity resonator, the temperature of the slit or the temperature of the specimen is preferably installed. Moreover, the data processing device 44 is preferably further provided with a correction means 46 that corrects the measured resonance peak level value based upon the temperature dependency stored in the temperature dependency value storing unit 42 by using the detected temperature from the temperature sensor 40.

Example 1

Referring to a flow chart shown in Fig. 14, the following description will discuss measurements of a specimen having a thickness "t" that is a known fixed value.
At step 1, in a state without a specimen (blank), a resonance peak level $P_0$ is preliminarily measured.
Next, at step 2, a resonance peak level $P_S$ of the specimen is measured.
At step 3, the difference $\Delta P(= P_0 - P_S)$ between the two values is calculated. This $\Delta P$ is proportional to a value $\varepsilon" \cdot t$ obtained by multiplying the dielectric loss factor $\varepsilon"$ of the specimen by the thickness "t" of the specimen. Therefore, with respect to specimens having the same thickness "t", the relationship between the moisture content and $\Delta P$ has been preliminarily prepared as a calibration line; thus, based upon the value of $\Delta P$ obtained at step 3, the moisture percentage can be obtained. For example, a specimen is moistened under environments of three conditions or more having different absolute humidity, and the moisture percentage (weight %) and the value of $\Delta P$ are measured under the respective conditions so that a calibration line that indicates the relationship between the two factors is preliminarily prepared.
At step 4, the value of $\Delta P$, obtained at step 3, is applied to the above-mentioned calibration line to determine a moisture percentage. In the case of on-line measurements, steps 2 to 4 are repeated at the same rate of time.
In Examples 1-1 to 1-3 as well as in Comparative Examples 1 and 2, cavity resonators of five types having different resonance frequencies in a range of 1 to 30GHz were used so that specimens (coated films each including one coat layer) were measured. The results are shown in Table 2.

[Table 2]

|  | Resonance frequency of wave guide | Peak level (dB) | | Applicability to coater to |
|---|---|---|---|---|
|  |  | With specimen | Blank |  |
| Example 1 - 1 | 4 GHz | -11.50 | -7.78 | ○ |
| Example 1 - 2 | 12 GHz | -36.01 | -33.64 | ○ |
| Example 1 - 3 | 19 GHz | -53.21 | -52.02 | ○ |
| Comparative Example 1 | 0.5 GHz | Unmeasurable | | × |
| Comparative Example 2 | 30 GHz | -68.23 | -68.21 | ○ |

The above results indicate that in the case of a resonance frequency of 30GHz, a certain measurement is available. However, a measuring area becomes smaller due to the smaller dimension of a resonator at a frequency beyond the frequency to cause a reduction in the measuring sensitivity, and a centering adjustment, which is carried out to allow the paired resonators to be aligned face to face in parallel with each other, becomes difficult as well as the price of the oscillators becomes more expensive due to the shorter wavelength. Therefore, this frequency is not practical in use. Moreover, in the case of a frequency of 0.5GHz or less, since the dimension of a resonator becomes greater beyond a level of several tens of centimeters, and since the dielectric loss factor of water also becomes smaller, it is difficult to carry out practical measurements. The appropriate measuring frequency is in a range from 1 to 25 GHz.

Example 2

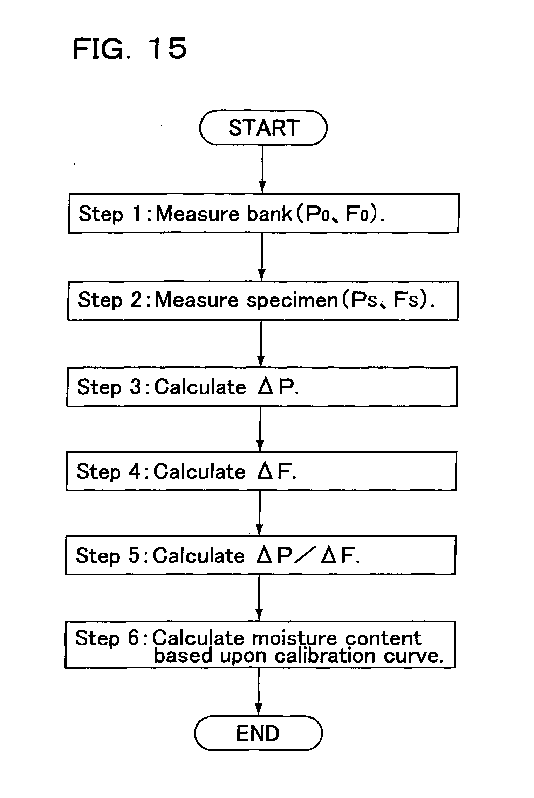

Referring to a flow chart shown in Fig. 15, the following description will discuss a method of measuring a specimen having a thickness that is not fixed.

At step 1, in a state without a specimen (blank), a resonance peak level $P_0$ and a resonance frequency $F_0$ are preliminarily measured.
Next, at step 2, a resonance peak level $P_S$ and a resonance frequency $F_S$ of a specimen are measured.

At step 3, a difference $\Delta P (= P_0 - P_S)$ in peak levels between the blank state and the specimen is calculated.

At step 4, a difference $\Delta F$ in resonance frequencies between the blank state and the specimen is calculated. In the cavity resonator, the dielectric constant $\varepsilon'$ is represented by the following equation (1):

$$\varepsilon' - 1 = K1 \times \Delta F / t \qquad (1)$$

Here, as described earlier, since $\Delta P$ is proportional to a value $\varepsilon'' \cdot t$, the following equation (2) is obtained:

$$\varepsilon'' = K2 \times \Delta P / t \qquad (2)$$

Here, K1 and K2 are respectively device constants.
When "t" is eliminated from the equations (1) and (2), the following equation (3) is obtained:

$$\varepsilon'' = K2/K1 \cdot (\varepsilon' - 1) \times \Delta P / \Delta F \qquad (3)$$

When the dielectric constant $\varepsilon'$ is constant, equation (3) is represented by equation (4):

$$\varepsilon'' = K3 \times (\Delta P / \Delta F) \qquad (4)$$

Here, K3 is a constant
In other words, in the case of when the quantity of moisture is small with respect to the specimen film, that is, $\varepsilon'$ is constant, $\varepsilon''$ is proportional to $\Delta P / \Delta F$, independent of the thickness "t" of the specimen. Therefore, $\Delta P / \Delta F$, obtained at step 5, forms a value that is correlated with the moisture percentage. When a calibration line is preliminarily prepared in the same manner as Example 1, the moisture content is found from $\Delta P / \Delta F$ through this calibration line (step 6).

Example 3

The following description will discuss a method by which, with respect to a specimen such as a coated film, having a single coat layer or multiple coat layers formed on one face or both of the faces of a film serving as a base substrate, the moisture content of each of the layers is found.

(Measurement on $\Delta \varepsilon''$ of coat layer)

A specimen prepared by forming a first coat layer on a PET film and an uncoated PET film were moistened under normal temperature and normal humidity and each of dielectric loss factors $\varepsilon''_{wet}$ under a state in which moisture had reached an equilibrium state were measured by using a molecular orientation meter. Based upon the relationship in dielectric loss factors between the specimen bearing the first coat layer and the uncoated film, the dielectric loss factor $\varepsilon''_{first\ coat\ layer\ wet}$ under normal temperature and normal humidity with respect to the first coat layer was obtained. Next, the dielectric loss factor $\varepsilon''_{dry}$, in a state in which moisture had been sufficiently removed was measured by using a molecular orientation meter so that the dielectric loss factor $\varepsilon''_{first\ coat\ layer\ dry}$ in the state in which moisture had been removed was obtained in the same manner. Here, a difference $\Delta \varepsilon''_{first\ coat\ layer}$ between the two factors corresponds to a moisture content (that is, moisture percentage) per unit volume of the first coat layer.
By carrying out the same processes, the dielectric loss factors $\Delta \varepsilon''_{second\ coat\ layer}$, $\Delta \varepsilon''_{thrid\ coat\ layer}$, $\cdots$ of the second, third, $\cdots$ coat layers are obtained. These values are inherent physical values measured under normal temperature and normal humidity conditions.

(Measurements of thickness)

The thickness $t_{base}$ of a base film, the thicknesses of respective coat layers $t_{first\ coat\ layer}$ $t_{second\ coat\ layer}$,

$t_{third\ coat\ layer}$, ···, and the total thickness $t_{total}$ are preliminarily measured through a thickness meter, amounts of respective coat layers and the like.

(Calculation of moisture percentage on respective coat layers)

Referring to Fig. 16, the following description will discuss a sequence of measurements.

At step 1, in a state without a specimen (blank), a resonance peak level $P_0$ is preliminarily measured.
At step 2, a resonance peak level $P_S$ of a specimen constituted by coated films having one layer or a plurality of layers is measured.

At step 3, a difference $\Delta P_{total}(= P_0 - P_S)$ in the two peak levels is calculated.
Here, the following description will discuss the rate of distribution of moisture content contained in the base film and respective coat layers to the total moisture content contained in the entire coated films. When moisture among the coat layers has reached an equilibrium state, supposing that the total moisture content is represented by "W", the following equation (5) is obtained by using $\Delta\varepsilon''$ and the thickness t of each of the layers.

$$W = \Delta\varepsilon''_{base} \cdot t_{base} + \Delta\varepsilon''_{first\ coat\ layer} \cdot t_{first\ coat\ layer} + \Delta\varepsilon''_{second\ coat\ layer} \cdot t_{second\ coat\ layer} +$$
$$\Delta\varepsilon''_{third\ coat\ layer} \cdot t_{third\ coat\ layer} + \cdots \quad (5)$$

Therefore, the rate of distribution $R_X$ to each of the layers is indicated by the following equation (6):

$$R_X = \Delta\varepsilon''_X \cdot t_X / W \qquad (6)$$

Here, X represents the base film or any one of the coat layers.

At step 4, by using the above-mentioned sequence of processes, the rate of moisture distribution for each of the layers is obtained based upon $\Delta\varepsilon''$ and thickness "t" of each of the layers.
Moreover, since $\Delta P_{total}$, is proportional to the total moisture content of the coated film, the moisture content of each of the layers is indicated by a value that is in proportion to $\Delta P_X$ found by the following equation (7)(step 5):

$$\Delta P_X = \Delta P_{total} \times R_X \qquad (7)$$

Therefore, $\Delta P_X/t_X$ forms a value that is correlated with the moisture percentage of each of the layers (step 6).
In the case of on-line measurements, steps 2 to 6 are repeated at the same rate of time.

Example 4

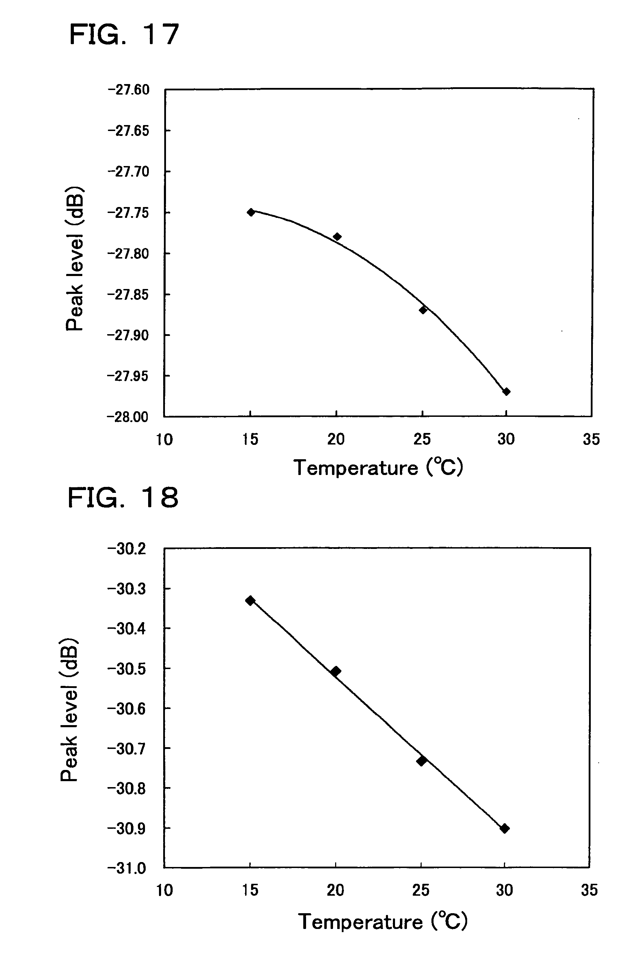

The following description will discuss a sequence of measuring processes upon conducting a temperature correction.
(Temperature correction in blank state)
A microwave cavity resonator is placed in a room which is temperature and humidity-controlled, and the room conditions are controlled to a desired environment After a sufficient period of time has been elapsed, measurements are carried out in the blank state (without using any specimen). Next, the temperature is raised by 5° C while the absolute humidity is maintained so as not to change. After a sufficient period of time has been elapsed, measurements are again carried out in the blank state. In the same manner, values in the blank state are measured after a further temperature rise of 5° C as well as after a temperature rise of 10° C. Fig. 17 is a graph that indicates fluctuations in the blank value obtained by setting the initial temperature to 15° C and by raising the temperature 5° C at a time. In this case, the absolute humidity is set to a constant value at 9.0 $g/m^3$. When this graph is second-order approximated by using a least square method, the following quadratic equation is obtained: $y = a_0 x^2 + box + c_0$. Here, $a_0$ and $b_0$ serve as coefficients used for carrying out temperature corrections, and a correction equation used for conducting temperature corrections is represented by the following equation (8). Here, the value obtained through this equation is a peak level (peak level after correction) upon conversion to a reference temperature X (° C).

$$P_0' = P_0 + a_0 \times (X - T_0)^2 \times b_0 \times (X - T_0) \qquad (8)$$

Here, $P_0'$ represents a peak level after correction, $P_0$ represents a peak level before correction, X represents a reference temperature, To represents a temperature of the wave guide, and $a_0$ and $b_0$ represent correction coefficients upon measurements in the blank state.

(Temperature correction upon measurements)

Under the same surrounding conditions, the resonance peak level $P_S$ of specimens to be measured and wave guide temperature $T_S$ are measured. Based upon the gradient of a graph in Fig. 18 drawn in the same manner as Fig. 17, correction coefficients $a_S$ and $b_S$ to be used upon measuring a specimen are found. Therefore, the correction equation used for the temperature correction upon measuring the specimen is represented by the following equation (9). Here, the value obtained through this equation is a peak level (peak level after correction) upon conversion to a reference temperature X (° C).

$$P_S' = P_S + a_S \times (X - T_S)^2 \times b_S \times (X - T_S) \qquad (9)$$

Here, $P_S'$ represents a peak level after correction, $P_S$ represents a peak level before correction, X represents a reference temperature, $T_S$ represents a temperature of the wave guide.

(Calculation of ∆P)

As described earlier, the difference ∆P in peak levels is indicated by ∆P = $P_0$ - $P_S$, and in the same manner, ∆P', obtained after the temperature correction, is indicated by ∆P' = $P'_0$ - $P_S'$.
The advantage of this correction is that, even under conditions in which temperatures are extremely different, such as summer time and winter time, by determining a certain reference temperature, values can be compared as those obtained at the reference temperature. Moreover, this correction is particularly effective in the case when, after carrying out the blank measurement, continuous on-line measuring processes are carried out In other words, even in the case when, with respect to the wave guide temperature obtained in the blank measurement, the wave guide temperature either rises or drops gradually during on-line measurements, by detecting the wave guide temperature at that point of time, the peak level at the reference temperature is calculated from equation (8), without having to newly measure the peak level at the time of the blank measurement, and with respect to the peak level at the time of the specimen measurement, the peak level at the reference temperature is simultaneously calculated based upon equation (9) by detecting the wave guide temperature. In this case, a means used for successively detecting the wave guide temperature may be installed (for example, a thermocouple is bonded to the wave guide side face) and a program which conducts the above-mentioned calculations is installed in the computer so that upon on-line measurements, the value of ∆P is measured instantaneously at a desired reference temperature. Alternatively, the temperature in the vicinity of the slit and the temperature of the specimen may be measured in place of the wave guide temperature. The temperature of the specimen or the like is preferably measured by using a non-contact temperature detector.
In the case when the moisture content is on-line measured, by utilizing the results, feed-back controlling operations can be carried out to adjust the moisture content of a coating solution for use in forming a coat layer and also to adjust the drying conditions of the coat layer, for example, adjustments on the drying temperature of a drier, the amount of air flow, etc.

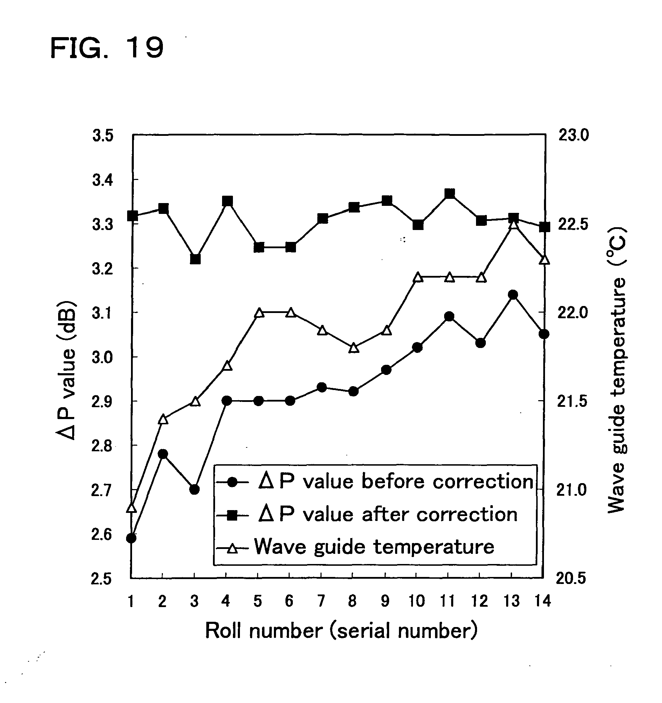

Fig. 19 shows the wave guide temperature and ∆P values before and after the temperature correction with respect to 14 specimens (in which specimen numbers are indicated on the axis of abscissa as roll numbers (serial number)) of the same kind that have been on-line measured. After the temperature correction, the ∆P values are maintained within a virtually fixed range of values so that the effects of the temperature correction are clearly exerted.

INDUSTRIAL APPLICABILITY

The present invention makes it possible to measure the moisture content or moisture percentage of a sheet-shaped material such as a coated film in which a film is used as a base substrate, with a coat layer being formed on the film, and is also utilized for measurements on the dried state of a coat layer during a printing process. Moreover, the present invention is easily applied to an on-line system, and utilized for process managements such as printing processes.

BRIEF DESCRIPTIONS OF THE DRAWINGS

Fig. 1 is a schematic structural drawing that shows one embodiment of a moisture content measuring device.

Fig. 2 is a schematic structural drawing that shows a first example of a microwave cavity resonator used in the present invention.

Fig. 3 is a schematic structural drawing that shows a second example of a microwave cavity resonator used in the present invention.

Fig. 4 is a graph that shows a resonance curve obtained by resonance of the microwave cavity resonator.

Fig. 5 is a drawing that indicates an equivalent circuit diagram and a resonance curve, which explain a principle of resonance in the microwave cavity resonator.

Fig. 6 is a block diagram that shows a structure of the embodiment

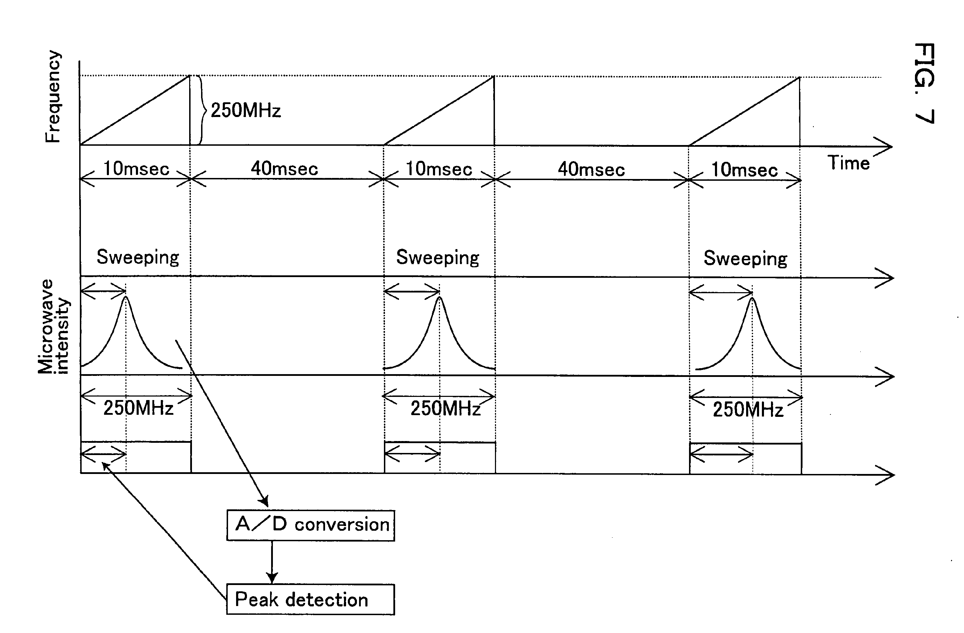

Fig. 7 is a time chart that shows operations of the embodiment

Fig. 8 is a schematic structural drawing that shows one embodiment of a moisture content measuring device of an on-line type.

Figs. 9(A) and 9(B) are perspective views that show a state in which guides are attached to an end portion on the slit side of a wave guide.

Fig. 10(A) is a perspective sectional view of a rectangular wave guide; Figs. 10(B) to 10(D) are drawings that show electromagnetic field distributions inside a cavity resonator in the case of $TE_{103}$ mode; Fig. 10(B) is a sectional view showing an XY face; Fig. 10(C) is a sectional view showing a YZ face; and Fig. 10(B) is a sectional view showing an XZ face.

Fig. 11 is a front view that shows a state in which a cavity resonator 1 is allowed to move.

Figs. 12 are schematic structural drawings that show the entire measuring system of the embodiment Fig. 12(A) shows a save position; and Fig. 12(B) shows a measuring position.

Fig. 13 is a block diagram that shows a part used for carrying out a temperature correcting process.

Fig. 14 is a flow chart that shows one example of operations in the embodiment

Fig. 15 is a flow chart that shows another example of operations in the embodiment

Fig. 16 is a flow chart that shows the other example of operations in the embodiment

Fig. 17 is a graph that shows a temperature dependency in a blank state.

Fig. 18 is a graph that shows a temperature dependency upon measuring a specimen.

Fig. 19 is a graph that shows the results of temperature corrections.

EXPLANATIONS OF NUMERALS

1 Cavity resonator
2A, 2B Wave guide
4a, 4b Resonator portion of wave guide
6a, 6b, 16a, 16b Traveling-wave portion of wave guide portion
8a, 8b Iris plates
10 Specimen
12 Slit
14a, 14b Antenna
20 Microwave sweep oscillator
22 Detector
24 Amplifier-A/D converter
30 Peak level detector
32 Resonance frequency detector
34 Operation unit
40 Temperature sensor
42 Temperature dependency value storage unit
44 Data processing unit
46 Correction means

**Claims**

1. A method of measuring moisture content, comprising the following:

   using a microwave cavity resonator that is provided with two holed iris plates which are arranged vertically in the direction of a tube axis at mid points of a wave guide, a portion between the iris plates forming a resonator portion and the outside of each of the iris plates forming traveling wave portions, and with a slit in which a specimen is disposed being placed in a manner so as to cross the resonator portion;
   setting a measuring frequency in a predetermined range between 1 to 25 GHz; and
   measuring the moisture content or the moisture percentage of the specimen based upon a difference in resonance peak level between cases when the specimen is not present in the slit and the specimen is present in the slit

2. The method of measuring moisture content according to claim 1, wherein the moisture percentage of the specimen is determined based upon a value obtained by dividing the difference in resonance peak levels between the presence and absence of the specimen by a difference in resonance frequency between the presence and absence of the specimen.

3. The method of measuring moisture content according to claim 1, wherein:

   measurements are carried out on a specimen of a film substrate bearing a coat layer on the surface thereof as well as on a specimen of a film substrate without the coat layer, and
   the moisture content or the moisture percentage of only the coat layer is determined by subtracting the measured value of the specimen without the coat layer from the measured value of the specimen with the coat layer.

4. The method of measuring moisture content according to claim 1, wherein:

   measurements are carried out on a specimen of a film substrate bearing a plurality of coat layers laminated on the surface thereof as well as on a specimen of a film substrate bearing coat layers except for the outermost coat layer of the coat layers; and
   the moisture content or the moisture percentage of only the outermost coat layer is determined by subtracting the measured value of the specimen without the outermost coat layer of the coat layers from the measured value of the specimen with the plurality of coat layers.

5. The method of measuring moisture content according to any of claims 1 to 4, wherein,
   measuring a temperature dependency of the resonance peak level preliminarily,
   detecting a temperature of the microwave cavity resonator, a temperature of the surroundings of the slit or a temperature of the specimen when measuring the specimen, and
   correcting the measured resonance peak level value based upon the temperature dependency by using the detected temperature.

6. A moisture content measuring device comprising:

   a microwave cavity resonator that is provided with two holed iris plates which are arranged vertically in the direction of a tube axis at mid points of a wave guide, a portion between the iris plates forming a resonator portion and the outside of each of the iris plates forming traveling wave portions, and with a slit in which a specimen is disposed being placed in a manner so as to cross the resonator portion;
   a microwave sweep oscillator which is connected to one of the pair of traveling wave portions and oscillates at a frequency in a predetermined range between 1 to 25 GHz;
   a microwave intensity receiver that is connected to the other of the pair of traveling wave portions; and
   a data processing device which, upon receipt of a signal from the microwave intensity receiver, detects a peak level, and determines the moisture content or the moisture percentage of a specimen based upon a difference in resonance peak level between cases when the specimen is not present in the slit and the specimen is present in the slit

7. The moisture content measuring device according to claim 6, wherein the microwave cavity resonator has the holes of the iris plates placed on the tube axes, with a diameter thereof being set to 1.0 to 20 mm.

8. The moisture content measuring device according to claim 6, wherein the resonator portion of the microwave cavity resonator has a dimension that is set to $TE_{10n}$ (n = 1, 2, 3, ...) in a resonance mode, with the slit being placed at a position that forms the maximum value in a electric field vector.

9. The moisture content measuring device according to any of claims 6 to 8, wherein:

one of the paired traveling wave portions of the microwave cavity resonator is constituted by a wave guide portion adjacent to one of the iris plates and another wave guide portion, connected to this wave guide portion, to which the microwave sweep oscillator is connected, and
the other traveling wave portion is constituted by a wave guide portion adjacent to the other iris plate and another wave guide portion, connected to this wave guide portion, to which the microwave intensity receiver is connected.

10. The moisture content measuring device according to any of claims 6 to 9, wherein both the wave guide to which the microwave sweep oscillator is connected and the wave guide to which the microwave intensity receiver is connected are prepared as a coaxial wave guide converter with a one-sided flange.

11. The moisture content measuring device according to any of claims 6 to 10, wherein,
the data processing device further detects a resonance frequency upon receipt of a signal from the microwave intensity receiver and determines the moisture percentage of the specimen based upon a value obtained by dividing the difference in resonance peak level between the presence and absence of the specimen by a difference in resonance frequency between the presence and absence of the specimen.

12. The moisture content measuring device according to any of claims 6 to 11, further comprising:

a specimen supplying mechanism that continuously supplies a specimen to the slit of the microwave cavity resonator,
wherein the data processing device further comprises a storage unit that stores a resonance peak level value when no specimen is present in the slit or a resonance frequency value in addition to the resonance peak level value, and determines a moisture content or a moisture percentage of the specimen by using a measured value upon presence of the specimen in the slit and a value upon absence of the specimen in the slit that is stored in the storage unit,
whereby an on-line measuring device that continuously measures the specimen is formed.

13. The moisture content measuring device according to claim 12, further comprising:

a temperature dependency value storage unit that stores a preliminarily measured temperature dependency of the resonance peak level; and
a temperature sensor which detects a temperature of the microwave cavity resonator, a temperature of the surroundings of the slit and a temperature of the specimen when measuring the specimen,
wherein, the data processing device further comprises a correction means that corrects the resonance peak level measured value based upon the temperature dependency stored in the temperature dependency value storage unit by using the detected temperature from the temperature sensor.

14. The moisture content measuring device according to claim 12 or claim 13, wherein a guide that has a shape for guiding the specimen to the slit is attached to an end portion of the slit side on the outside face of an E face of the wave guide of the microwave cavity resonator.

15. The moisture content measuring device according to any of claims 12 to 14, further comprising

proximity sensors that are placed in the vicinity of the slit at opposing positions with respective gaps to the surface and rear face of the specimen that passes through the slit; and
a fluctuation detecting unit that detects a fluctuation of the specimen passing through the slit based on a detected values of the proximity sensors.

16. The moisture content measuring device according to claim 15, further comprising

a save mechanism that keeps away the microwave cavity resonator from the specimen when a fluctuation width detected by the fluctuation detecting unit exceeds a predetermined reference value.

## FIG. 1

14a

20

6a(16a)

Microwave sweep
oscillator

8a

4a

12

10

4b

8b

6b(16b)

24

Amplifier,
A/D convertor

14b

22

## FIG. 2

## FIG. 3

# FIG. 4

Resonance curve

Blank

with specimen

$\varepsilon'$

$\varepsilon''$

Frequency

Microwave permeation intensity

FIG. 5

$Q = R/\omega_0 L$

$\omega_0 L = 1/\omega_0 C$

$\omega_0 =$ Resonance angular frequency

$Q = \omega_0 / 2\triangle\omega$

EP 1 655 601 A1

# FIG. 6

20 — Microwave sweep oscillator

1 — Cavity resonator

22,24 — Microwave intensity receiver

30 — Peak level detector

32 — Resonance frequency detector

34 — Operation unit

# FIG. 7

# FIG. 8

# FIG. 9

(A)

30a

E face

2

H face

12

(B)

30b

H face

2

E face

12

# FIG. 10

(A)

H face

Y

Z

2

X

E face

(B)

Y

Z⊗ →X

2

Ey

(C)

Y

X⊙ →Z

2

Ey

(D)

X

Y⊙ →Z

2

Hx  Hz

## FIG. 11

## FIG. 12

## FIG. 13

## FIG. 14

START

Step 1 : Measure blank ($P_0$).

Step 2 : Measure specimen ($P_s$).

Step 3 : Calculate $\Delta P$.

Step 4 : Calculate moisture percentage based upon calibration curve.

END

# FIG. 15

START

Step 1 : Measure bank $(P_0, F_0)$.

Step 2 : Measure specimen $(P_s, F_s)$.

Step 3 : Calculate $\Delta P$.

Step 4 : Calculate $\Delta F$.

Step 5 : Calculate $\Delta P \diagup \Delta F$.

Step 6 : Calculate moisture content based upon calibration curve.

END

## FIG. 16

$$\boxed{\text{START}}$$

Step 1 : Measure blank $(P_0, F_0)$.

Step 2 : Measure specimen $(P_s, F_s)$.

Step 3 : Calculate $\Delta P_{total}$.

Step 4 : Calculate moisture distribution rate $R_x$.

Step 5 : $\Delta P_x = \Delta P_{total} \times R_x$

Step 6 : Moisture percentage of each layer $\Delta P / t_x$

$$\boxed{\text{END}}$$

## FIG. 17

## FIG. 18

## FIG. 19

EP 1 655 601 A1

**INTERNATIONAL SEARCH REPORT**

| International application No. |
| --- |
| PCT/JP2004/010957 |

| A. CLASSIFICATION OF SUBJECT MATTER |
| --- |
| Int.Cl$^7$  G01N22/04 |

According to International Patent Classification (IPC) or to both national classification and IPC

| B. FIELDS SEARCHED |
| --- |
| Minimum documentation searched (classification system followed by classification symbols)<br>Int.Cl$^7$  G01N22/00-22/04 |

Documentation searched other than minimum documentation to the extent that such documents are included in the fields searched

| Jitsuyo Shinan Koho | 1922-1996 | Toroku Jitsuyo Shinan Koho | 1994-2004 |
| --- | --- | --- | --- |
| Kokai Jitsuyo Shinan Koho | 1971-2004 | Jitsuyo Shinan Toroku Koho | 1996-2004 |

Electronic data base consulted during the international search (name of data base and, where practicable, search terms used)
JICST FILE (JOIS)

C. DOCUMENTS CONSIDERED TO BE RELEVANT

| Category* | Citation of document, with indication, where appropriate, of the relevant passages | Relevant to claim No. |
| --- | --- | --- |
| Y<br>A | JP 62-195568 A  (Kanzaki Paper Mfg. Co., Ltd.),<br>28 August, 1987 (28.08.87),<br>Full text; all drawings<br>(Family: none) | 1,2,5-14<br>3,4,15,16 |
| Y<br>A | JP 56-39447 A  (Shin'ichi SASAKI),<br>15 April, 1981 (15.04.81),<br>Full text; all drawings<br>(Family: none) | 1,2,5-14<br>3,4,15,16 |
| Y<br>A | JP 9-325123 A  (Hauni Maschinenbau A.G.),<br>16 December, 1997 (16.12.97),<br>Full text; all drawings<br>& EP 791823 A2      & US 6163158 A<br>& DE 19705260 A     & CN 1213080 A | 1,2,5-14<br>3,4,15,16 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>04 October, 2004 (04.10.04) | Date of mailing of the international search report<br>19 October, 2004 (19.10.04) |
| --- | --- |
| Name and mailing address of the ISA/<br>Japanese Patent Office | Authorized officer |
| Facsimile No. | Telephone No. |

Form PCT/ISA/210 (second sheet) (January 2004)

INTERNATIONAL SEARCH REPORT

International application No.

PCT/JP2004/010957

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | JP 49-11195 A  (Yoshio KOBAYASHI),<br>31 January, 1974 (31.01.74),<br>Full text; all drawings<br>(Family: none) | 1,2,5-14<br>3,4,15,16 |
| Y | JP 63-7892 Y2  (New Japan Radio Co., Ltd.),<br>08 March, 1988 (08.03.88),<br>Full text; all drawings<br>(Family: none) | 2 |
| Y | JP 2-55745 B2  (N.V. Philips'<br>Gloeilampenfabrieken),<br>28 November, 1990 (28.11.90),<br>Full text; all drawings<br>& US 4361801 A          & DE 2928487 A<br>& FR 2461945 A          & CH 650862 A | 2 |
| Y | JP 60-135752 A  (Yokokawa Hokushin Denki<br>Kabushiki Kaisha),<br>19 July, 1985 (19.07.85),<br>Full text; all drawings<br>(Family: none) | 5,13 |
| Y | JP 11-23492 A  (Yugen Kaisha Akkusu),<br>29 January, 1999 (29.01.99),<br>Full text; all drawings<br>(Family: none) | 12-14 |
| A | JP 2-26182 B2  (Honshu Seishi Kabushiki Kaisha),<br>07 June, 1990 (07.06.90),<br>Full text; all drawings<br>(Family: none) | 3 |
| A | JP 2000-356601 A  (Meidensha Corp.),<br>26 December, 2000 (26.12.00),<br>Full text; all drawings<br>(Family: none) | 10 |

Form PCT/ISA/210 (continuation of second sheet) (January 2004)